# EUROPÄISCHE PATENTSCHRIFT

(11) **EP 0 963 558 B1**
(45) Veröffentlichungstag und Bekanntmachung des Hinweises auf die Patenterteilung: **29.10.2003**
(21) Anmeldenummer: 98912365.8
(22) Anmeldetag: 21.02.1998
(51) Int. Cl.: G01R 29/10, H05K 9/00

(54) **KUNSTSTOFFKÖRPER, INSBESONDERE FLÄCHENELEMENT, ZUR EINSCHRÄNKUNG VON HF-REFLEXIONEN**
PLASTIC BODY, IN PARTICULAR FLAT ELEMENT, FOR LIMITING HF REFLECTION
CORPS EN MATIERE PLASTIQUE, NOTAMMENT ELEMENT PLAT, POUR LIMITER LES REFLEXIONS DE HAUTE FREQUENCE

(30) Priorität: 01.03.1997 DE 29703725 U
(43) Veröffentlichungstag der Anmeldung: 15.12.1999
(73) Patentinhaber: Hoffmann, Peter, 58239 Schwerte (DE)
(72) Erfinder: Hoffmann, Peter, 58239 Schwerte (DE)
(74) Vertreter: Patentanwälte Meinke, Dabringhaus und Partner
(86) Internationale Anmeldenummer: EP9801013
(87) Internationale Veröffentlichungsnummer: WO98038518

(56) Entgegenhaltungen:
- EP-A- 0 301 561
- EP-A- 0 398 672
- EP-A- 0 437 979
- EP-A- 0 470 262
- EP-A- 0 550 373
- WO-A-93/09944
- DE-A- 3 703 755
- FR-A- 2 675 654
- US-A- 5 278 377

## Beschreibung

Die Erfindung richtet sich auf einen Kunststoffkörper, insbesondere Flächenelement, zur Einschränkung von Reflexionen hochfrequenter Strahlung in HF-geschirmten Bereichen, wie Aufstellungsorten von Computern, HF-geschirmten Räumen od. dgl., insbesondere bei Meßvorgängen an hochfrequent-strahlenden Objekten, wobei im Kunststoff elektrisch leitende bzw. hochfrequente Strahlen streuende Partikel vorgesehen sind. Eine Folie bzw. ein entsprechend beschichtetes Gewebe ist aus der WO 93/09944-A bekannt.

Um zu vergleichsweise genauen Meßergebnissen in Verbindung mit der Überprüfung von hochfrequent-strahlenden Objekten zu gelangen, werden diese in Meßräumen überprüft, deren Wände mit einer die hochfrequente Strahlung nicht oder nur in äußerst geringem Maße reflektierenden Beschichtung versehen sind. Bei einer derartigen Beschichtung handelt es sich in der Regel um Ferrit-Kacheln, die in der Lage sind, auftreffende Hochfrequenz (im folgenden HF)-Strahlen zu absorbieren. Die Ferrit-Kacheln werden auf die einen. Faradaykäfig und damit die HF-Abschirmung bildenden Metallwände des Prüfraumes aufgeklebt oder in anderer Weise dort befestigt. Eine solche Beschichtung ist nicht nur relativ schwer, was die Statik derartiger Räume besinflußt, sie ist auch vergleichsweise teuer.

Eine Abschirmvorrichtung zur Abschirmung gegen statische elektrische Felder als Mehrschichtfolie ist aus der DE-37 07 238-A1 bekannt, dort ist in Kunststoffschaum ein Pulver aus leitfähigem Material eingebracht. Die gezielte Vermeidung einer elektrischen Quer- und Längsleitung durch den Kunststoff ist dieser Literaturstelle nicht zu entehmen. Abschirmplatten oder -folien sind in weiteren Literaturstellen vorbeschrieben, wobei auch besondere Einrichtungen zur Abschirmung vorbekannt sind, so die DT-20 26 890, die DE-Al-Publikationen 34 17 895, 39 00 856, 40 03 908, 40 26 403, 41 01 869, 42 43 368, 43 24 916, 43 35 626 oder die 195 18 541-C2. Zum Stand der Technik gehört auch ein Absorber für elektromagnetische Strahlen nach der DE-37 03 755-A1, ein elektromagnetische Wellen absorbierendes Material nach der FR-2 675 654-A1 bzw. der EP-0 437 979-A.

Den oben zitierten Literaturstellen ist in der Regel gemeinsam, daß dort versucht wird, elektrisch leitende Kunststoffe bereitzustellen, um die jeweils beschriebenen Vorteile zu erreichen. Dies ist bei der Erfindung anders, deren Aufgabe in der Schaffung einer Lösung besteht, mit der bei gleichem Dämpfungs- bzw. Abschirmungseffekt die entsprechende Raumauskleidung leichter und insbesondere preiswerter gestaltet werden kann.

Mit einem Kunststoffkörper, insbesondere einem Flächenelement, der eingangs bezeichneten Art wird diese Aufgabe durch die Merkmale des Auspruchs 1 gelöst.

Weitere Ausgestaltungen der Erfindung ergeben sich aus der weiteren Beschreibung sowie aus den Unteransprüchen.

Vorteilhaft ist es, wenn es sich bei einem Teil der elektrischen Partikel im wesentlichen um Metallpulver, insbesondere Eisenpulver, handelt, bei einem anderen Teil kann es sich um Graphitpulver handeln.

Neben diesen Partikeln ist es erfindungsgemäß auch möglich, die Partikel aus Mangan, Zink und/oder Nickel auszubilden, wobei eine besonders vorteilhafte Ausgestaltung der Erfindung darin besteht, daß zur diffusen Streuung innerhalb des Materiales die eingebetteten elektrischen Partikel eine kristalline Struktur aufweisen.

Dies kann beispielsweise erfindungsgemäß dadurch erreicht werden, daß das Metallpulver ber seiner Herstellung mit Wasser verdüst und nicht in einer Schutzgasatmosphäre ausgetragen wird, so daß die wasserversprühten Partikel kristalline Oberflächen aufweisen gegenüber den schutzgasversprühten Partikeln, die in der Regel eine der Kugelform sehr nahekommende Form haben und diffuse Reflexionen nicht mehr zulassen, wie dies nach der Erfindung erwünscht ist.

Von der Gesamtmenge der eingebetteten Partikel als Zuschlagstoffe im Kunststoff kann das Eisenpulver bis zu 90 % betragen. Bei dem Rest kann es sich, wie oben schon angeführt, beispielsweise um Mangan-, Zink- und/oder Nickelstäube handeln, wobei an dieser Stelle ausdrücklich bemerkt sei, daß die Erfindung auf diese Mengenangaben nicht beschränkt ist, die Verteilung der Menge innerhalb des Kunststoffes kann auch in anderer Weise vorgenommen werden.

Besonders preiswert ist ein entsprechendes Flächenelement dann, wenn beispielsweise als Träger-Kunststoff Polyurethan vorgesehen ist. Hier kann es sich natürlich auch um Kunststoff-Harze od. dgl. handeln, wenn dies aus bestimmten Gründen gewünscht wird. Polyurethan hat die mit diesem Kunststoff verbundenen besonderen Vorteile, es ist vom Gewicht her leicht; einfach zu verarbeiten und insbesondere einfach in der Formgebung:

Die Flächenelemente können erfindungsgemäß in Plattenform ausgebildet sein, aber auch als Bänder oder Rollenware oder als geometrisch vergleichsweise kleine Platten, d.h. Fliesengröße aufweisen.

Ein Vorteil der Erfindung besteht darin, daß ein entsprechender Kunststoffkörper eine hohe Spannungsfestigkeit (> 10 kV/mm) aufweist, wobei die elektrisch leitenden Partikel vollständig eingeschlossen sind, so daß sie ihre dreidimensionalen Eigenschaften voll zur Wirkung bringen können. Eingesetzter hochreiner Kohlenstoff absorbiert in die Tiefe dringende hochfrequente Strahlungen, hier durch Umsetzung in Wärme. Die. Abstraktionsfähigkeit liegt bei > 20 dBµV im Mittel und einer Wirksamkeit von 15 MHz bis in den hohen GHz-Bereich.

Durch den Einsatz von Polyurethan-Hartschaum; der überwiegend eine definierte Bläschengröße ausbildet, ergibt sich eine hohe Wirksamkeit bei Frequenzen oberhalb 600 MHz, wobei erfindungsgemäß beim Herstellungsverfahren dieser Vorgang in Hochdruckformen unterschiedlicher Größen noch be-einflußt und gesteuert werden kann.

Gegenüber bekannten Ferrit-Werkstoffen hat ein Flächenelement nach der vorliegenden Erfindung einige Vorteile:
- 1 cm³ wiegt weniger als 0,2 gr (Ferrit 5,0 gr/cm³). Durch das niedrige Gewicht werden auf dem Transport keine Geräte beschädigt;
- es ist nicht leitfähig und kann somit als Dämpfungsmaterial ohne Isolation an spannungsführende Elemente gebracht werden;
- es ist montagefreundlich und umweltneutral;
- die Herstellung kann mit einfachsten Mitteln erfolgen;
- es kann in Absorberhallen zur Reflektionsdämpfung und in elektrischen und elektronischen Geräten zur Entstörung eingesetzt werden;
- durch das niedrige Gewicht werden Transportkosten gesenkt.

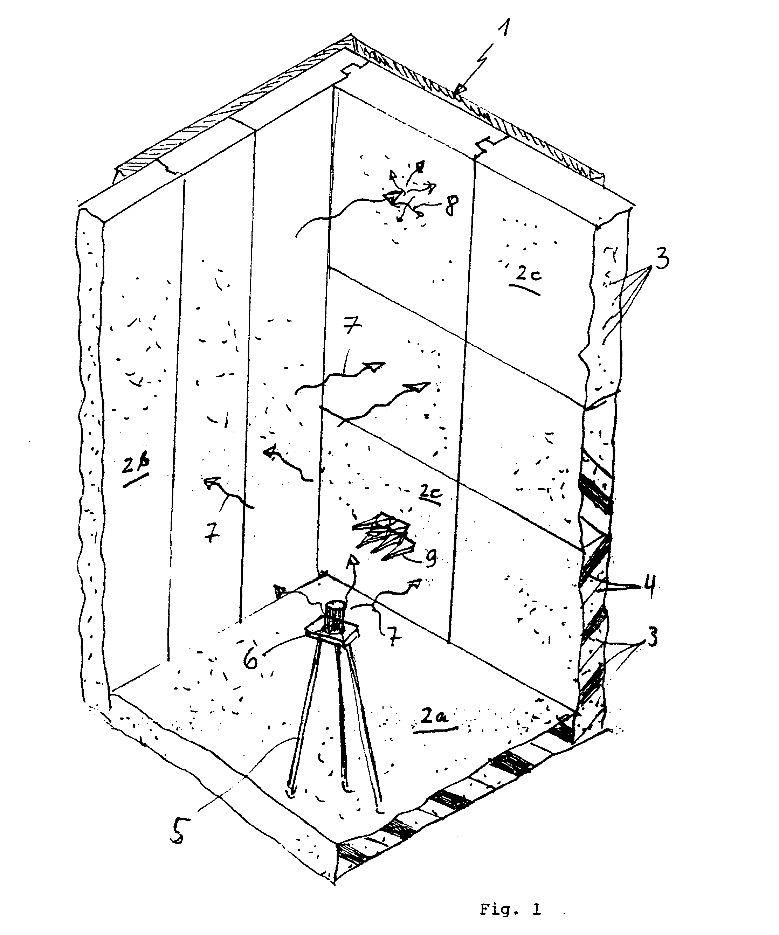

Die Erfindung ist nachstehend anhand der Zeichnung beispielsweise näher erläutert. Diese zeigt in der einzigen Figur in vereinfachter Darstellung die Raumecke eines Abschirmraumes.

Im dargestellten Beispiel ist die allgemein mit 1 bezeichnete Raumecke einer Metallabschirmung mit erfindungsgemäßen Flächenelementen zum Rauminneren hin ausgekleidet, wobei drei Varianten, ohne daß die Erfindung darauf beschränkt wäre, hier dargestellt sind, nämlich eine vergleichsweise großflächige Bodenplatte 2a, Bänder- bzw. Rollenflächenelemente 2b auf einer Wandseite und fliesenförmige Flächenelemente 2c auf der anderen Wandseite, die hier lediglich als Beispiel dienen.

Durch Punktierung sollen die elektrisch leitenden Partikel 3 in den Platten dargestellt sein, durch eine Kunststoff-Schraffierung 4 soll erkennbar sein, daß es sich um im Kunststoff eingebettete Partikel 3 handelt.

Angedeutet ist auf der Bodenplatte 2a ein, Stativ 5 mit einem HF-strahlenden Prüfling 6, wobei die ausgehenden HF-Strahlen mit gewellten Pfeilen 7 angedeutet sind. Im oberen Bildbereich ist das Auftreffen einer solchen HF-Strahlung auf Partikel angedeutet, wobei durch kleine Pfeile im Platteninneren eine diffuse Reflexionsstrahlung angedeutet sein soll.

Schließlich ist auf einer Fliese 2c noch die Möglichkeit angedeutet, Reflexionspyramiden 9 vorzusehen, die aber bekannt sind und die keine Besonderheit der Erfindung darstellen.

Ein Beispiel zur Herstellung sel wie folgt angegehen:

In die nicht aktive Komponente des Polyurethans werden Beimischungen (20 kg Kohlenstoff, 20 kg Eisenmangan/m³; es finden auch Verwendung Eisen, Nickel, Zink, je nach Frequenzgängigkeit) gerührt zur homogenen Masse und danach in eine druckdichte stabile Form verbracht, in der das Material expandieren kann. Das fertige Material kann mit einer Säge auf jegliche gewünschte Größe und Form geschnitten werden.

Natürlich sind die beschriebenen Ausführungsbeispiele der Erfindung noch in vielfacher Hinsicht abzuändern, ohne den Grundgedanken zu verlassen. So ist die Erfindung weder auf die besondere räumliche Gestaltung der einzelnen Flächen-elemente beschränkt, auch nicht auf die Art des Zusammenfügens über Nut oder Federn od. dgl. oder auf bestimmte metallische. Stäube, die in Kunststoff eingebettet sind, ebensowenig wie auf bestimmte Kunststoffe.

## Patentansprüche

1. Elektrich isolierender Kunststoffkörper, zur Einschränkung von. Reflexionen hochfrequenter Strahlung in HF-geschirmten Bereichen, wobei im Kunststoff elektrisch leitende bzw. hochfrequente Strahlen streuende Partikel vorgesehen sind,
**dadurch gekennzeichnet,**
**daß** die im Kunststoff (4) eingebetteten metallichen, Partikel (3) zur Streuung der elektromagnetischen Strahlung eine übereine Verdüsung mit Wasser erzeugte kristalline Struktur aufweisen und daß diese Partikel (3) jeweils vollständig im Kunststoff eingeschlossen sind.

2. Kunststoffkörper nach Anspruch 1,
**dadurch gekennzeichnet,**
**daß** es sich bei einem Teil der elektrischen Partikel um Eisenpulver handelt.

3. Kunststoffkörper nach Anspruch 1 oder 2,
**dadurch gekennzeichnet,**
**daß** es sich bei einem Teil der eingebetteten Partikel um Graphitpulver handelt.

4. Kunststoffkörper nach Anspruch 1
**dadurch gekennzeichnet,**
**daß** die eingebetteten Partikel (3) aus Mangan, Zink und/oder Nickel gebildet sind.

5. Kunststoffkörper nach einem der vorangehenden Ansprüche,
**dadurch gekennzeichnet,**
**daß** als Trägerkunststoff (2) Polyurethan od. dgl. vorgesehen ist.

6. Kunststoffkörper nach einem der vorangehenden Ansprüche,
**dadurch gekennzeichnet,**
**daß** der Kunststoffkörper als Flächenelement in Platten-(2a), Bänder-, Rollen- (2b) und/oder Fliesenform (2c) ausgebildet ist.

## Claims

1. An electrically insulating plastic body for limiting reflections of high-frequency radiation in HF-screened regions, wherein provided in the plastic material are particles which are electrically conductive or which scatter high-frequency rays, **characterised in that** the metallic particles (3) embedded in the plastic material (4) for scattering of the electromagnetic radiation are of a crystalline structure produced by way of spraying with water and that said particles (3) are each completely enclosed in the plastic material.

2. A plastic body according to claim 1 **characterised in that** a part of the electrical particles is iron powder.

3. A plastic body according to claim 1 or claim 2 **characterised in that** a part of the embedded particles is graphite powder.

4. A plastic body according to claim 1 **characterised in that** the embedded particles (3) are formed from manganese, zinc and/or nickel.

5. A plastic body according to one of the preceding claims **characterised in that** polyurethane or the like is provided as the carrier plastic material (2).

6. A plastic body according to one of the preceding claims **characterised in that** the plastic body is a flat element in the form of plates (2a), strips, rolls (2b) and/or fleeces (2c).

## Revendications

1. Corps en matière plastique électriquement isolant servant à limiter des réflexions d'un rayonnement à haute fréquence dans des zones protégées vis-à-vis des hautes fréquences, dans lequel des particules électriquement conductrices ou qui dispersent les rayonnements à hautes fréquences sont prévues dans la matière plastique,
**caractérisé en ce que** les particules métalliques (3) noyées dans la matière plastique (4) et servant à disperser le rayonnement électromagnétique possèdent une structure cristalline, produite au moyen d'une pulvérisation d'eau, et que ces particules (3) sont insérées respectivement entièrement dans la matière plastique.

2. Corps en matière plastique selon la revendication 1, **caractérisé en ce qu'**il s'agit de poudre de fer pour une partie des particules électriques.

3. Corps en matière plastique selon la revendication 1 ou 2, **caractérisé en ce qu'**il s'agit de poudre de graphite pour une partie des particules insérées.

4. Corps en matière plastique selon la revendication 1, **caractérisé en ce que** les parties insérées (3) sont formées de manganèse, de zinc et/ou de nickel.

5. Corps en matière plastique selon l'une des revendications précédentes, **caractérisé en ce qu'**il est prévu, comme matériau de la matière plastique de support (2), du polyuréthane ou analogue.

6. Corps en matière plastique selon l'une des revendications précédentes, **caractérisé en ce que** le corps en matière plastique est agencé sous la forme d'un élément de surface en forme de plaques (2a), de bandes, de rouleaux (2b) et/ou de carreaux (2c).
